# EUROPÄISCHE PATENTSCHRIFT

(11) **EP 1 910 265 B1**
(45) Veröffentlichungstag und Bekanntmachung des Hinweises auf die Patenterteilung: **25.02.2009**
(21) Anmeldenummer: 06754637.4
(22) Anmeldetag: 30.06.2006
(51) Int. Cl.: C07C 51/25, C07C 51/42, C07C 57/04, C08F 20/06, C08F 2/06, C08F 2/10

(54) **ANORDNUNG ZUR BEHANDLUNG EINES POLYMERISATIONSFÄHIGEN STOFFES**
ASSEMBLY FOR THE TREATMENT OF A POLYMERIZABLE MATERIAL
DISPOSITIF DE TRAITEMENT D'UNE SUBSTANCE POLYMERISABLE

(30) Priorität: 30.06.2005 DE 102005030416
(43) Veröffentlichungstag der Anmeldung: 16.04.2008
(73) Patentinhaber: Evonik Stockhausen GmbH, 47805 Krefeld (DE)
(72) Erfinder: MOSLER, Jürgen, 44577 Castrop-Rauxel (DE)
(74) Vertreter: Herzog, Martin
(86) Internationale Anmeldenummer: PCT/EP2006/006352
(87) Internationale Veröffentlichungsnummer: WO 2007/003358

(56) Entgegenhaltungen:
- DE-A1- 10 339 633
- US-A- 5 087 744
- US-A- 6 051 736
- US-B1- 6 498 272

## Beschreibung

Die vorliegende Erfindung betrifft eine Anordnung zur Behandlung eines polymerisationsfähigen Stoffes umfassend zumindest eine Begasungsvorrichtung zum Einleiten eines Gases in den polymerisationsfähigen Stoff und eine Heizvorrichtung zum Erhitzen des mit Gas versehenen polymerisationsfähigen Stoffes. Die Erfindung betrifft auch ein Verfahren zur Erwärmung eines polymerisationsfähigen Stoffes, eine Vorrichtung zur Herstellung von hochreiner (Meth)Acrylsäure sowie ein Verfahren zur Herstellung hochreiner (Meth)acrylsäure.

Unter (Meth)Acrylsäure wird vorliegend sowohl Methacrylsäure als auch Acrylsäure verstanden, wobei Acrylsäure besonders bevorzugt ist.

Es ist bekannt, dass polymerisationsfähige Stoffe wie (Meth)Acrylsäure und (Meth)Acrylsäureester etwa durch Wärme und/oder Einwirkung von Licht und/oder Peroxiden leicht zur Polymerisation gebracht werden können. Da jedoch bei der Herstellung, Aufarbeitung und/oder Lagerung die Polymerisation aus sicherheitstechnischen und wirtschaftlichen Gründen vermindert bzw. verhindert werden muss, besteht ein ständiger Bedarf an neuen, einfachen und wirksamen Methoden und Anlagen zur Verringerung der unkontrollierten Polymerisation. Weiter ist auch bekannt, dass die Polymerisation von (Meth)Acryläsure und (Meth)Acrylsäureestern durch Verwendung von Polymerisationsinhibitoren wie beispielsweise Hydrochinonen oder Hydrochinonderivaten zurückgedrängt werden kann. Auch die Gegenwart von Sauerstoff inhibiert die Polymerisation.

Üblicherweise wird (Meth)Acrylsäure durch katalytische Gasphasenoxidation ungesättigter Kohlenwasserstoffe wie beispielsweise Propylen oder Isobutylen hergestellt. Dabei wird zunächst ein gasförmiges Reaktionsgemisch enthaltend (Meth)Acrolein erhalten, welches in einer zweiten Oxidationsstufe unter Erhalt eines (Meth)Acrylsäure enthaltenden Produktgasgemisches oxidiert wird. Dieses Produktgasgemisch wird anschließend kondensiert oder in einem geeigneten Lösungsmittel absorbiert, wobei eine (Meth)Acrylsäure enthaltende flüssige Phase erhalten wird. Diese flüssige Phase, die neben der (Meth)Acrylsäure und gegebenenfalls dem Absorptionsmittel noch zahlreiche Nebenprodukte enthält, die bei der Gasphasenoxidation der ungesättigten Kohlenwasserstoffe als Ausgangsverbindungen gebildet werden, wird anschließend mittels weiterer Reinigungsverfahren, insbesondere mittels Destillation aufgereinigt. Gegebenenfalls wird die (Meth)Acrylsäure auch mit geeigneten Alkoholen zu den entsprechenden (Meth)Acrylsäureestern umgesetzt.

Bei der destillativen Aufreinigung von (Meth)Acrylsäure enthaltenden Flüssigkeiten oder aber bei der Veresterung müssen diese Flüssigkeiten jedoch auf eine für eine ausreichende Trennleistung bzw. für eine Veresterung erforderliche Temperatur erwärmt werden, wobei dieses Erwärmen vorzugsweise in geeigneten Rohrbündelwärmeaustauschem erfolgt. Um eine Polymerisation der (Meth)Acrylsäure während der thermisch belastenden Destillation bzw. der Veresterung zu Verhindern, ist es auch üblich, Polymerisationsinhibitoren zuzusetzen.

Die derzeit bekannten Anlagen zur Aufbereitung eines polymerisationsfähigen Stoffes können gerade im Bereich der Rohrbündelwärmetauscher noch keine ausreichende Sicherheit hinsichtlich auftretender Polymerisation bieten.

Es ist Aufgabe der vorliegenden Erfindung, die mit Bezug auf den Stand der Technik geschilderten technischen Probleme zumindest teilweise zu lindern. Insbesondere soll eine Anordnung vorgeschlagen werden, bei der eine Behandlung des polymerisationsfähigen Stoffes mit besonders geringer Polymerisation gewährleistet ist. Die Anordnung soll sich zudem durch einen einfachen Aufbau und geringen Wartungsaufwand auszeichnen. Schließlich sollen noch besonders geeignete Verfahren und Verwendungen dieser Anordnung sowie damit produzierter Produkte beschrieben werden.

Diese Aufgaben werden gelöst durch eine Anordnung zur Behandlung eines polymerisationsfähigen Stoffes mit den Merkmalen des Patentanspruchs 1, durch ein Verfahren zur Erwärmung eines polymerisationsfähigen Stoffes mit den Merkmalen des Anspruchs 8, durch eine Vorrichtung zur Herstellung von hochreiner (Meth)Acrylsäure mit den Merkmalen des Anspruchs 13 sowie durch ein Verfahren zur Herstellung hochreiner (Meth)Acrylsäure mit den Merkmalen des Anspruchs 14. Weitere vorteilhafte Ausgestaltungen der Anordnung, bevorzugte Betriebsverfahren sowie damit hergestellte Produkte werden in den abhängig formulierten Patentansprüchen beschrieben. Es ist darauf hinzuweisen, dass die in den Patentansprüchen einzeln aufgeführten Merkmale in beliebiger, technologisch sinnvoller, Weise miteinander kombiniert werden können und weitere Ausgestaltungen der Erfindung aufzeigen.

Die erfindungsgemäße Anordnung zur Behandlung eines polymerisationsfähigen Stoffes umfasst zumindest eine Begasungsvorrichtung zum Einleiten eines Gases in den polymerisationsfähigen Stoff und eine Heizvorrichtung zum Erhitzen des mit Gas versehenen polymerisationsfähigen Stoffes. Die Heizvorrichtung ist dabei so gestaltet, dass der polymerisationsfähige Stoff die Heizvorrichtung über einen Einlass entgegen der Schwerkraft durchströmt und Mittel zur gleichmäßigen Verteilung des Gases über den Einlass vorgesehen sind.

Der polymerisationsfähige Stoff liegt hier im wesentlichen als Flüssigkeit vor,
wobei das Gas mittels zumindest einer Begasungsvorrichtung in diese Flüssigkeit eingeleitet wird. Eine solche Begasungsvorrichtung kann jeweils eine einzelne Begasungsstelle aufweisen, es ist jedoch auch möglich, dass mehrere Begasungsstellen mittels einer einzelnen Begasungsvorrichtung verwirklicht werden. Im letzten genannten Fall ist es möglich, dass jede Begasungsstelle einzeln, in Gruppen oder alle zusammen betreibbar sind.

Die Heizvorrichtung ist mit Wärmeaustauschflächen ausgeführt, die mit dem polymerisationsfähigen Stoff in Kontakt gebracht werden. Die Heizvorrichtung kann auf jede bekannte Weise erhitzt werden, bevorzugt ist jedoch eine Erhitzung mit Wasserdampf. Die Heizvorrichtung weist getrennte Strömungswege für den Wasserdampf und den polymerisationsfähigen Stoff auf. Die Wärmeaustauschmedien (polymerisationsfähiger Stoff, Wasserdampf) können in jeder beliebigen Richtung zueinander geführt werden, insbesondere auch im Gegenstrom.

Mit Einlass ist insbesondere die Eintrittsöffnung der Heizvorrichtung gemeint. Der Einlass wird bevorzugt mit einem runden Querschnitt ausgeführt, der einen Durchmesser im Bereich von 500 bis 2500 mm hat. Der Einlass ist bevorzugt waagerecht angeordnet.

Die Anordnung ist nun so ausgestaltet, dass der polymerisationsfähige Stoff entgegen der Schwerkraft strömt. Bevorzugt strömt der polymerisationsfähige Stoff im wesentlichen parallel entgegen der Schwerkraft. Dies ist besonders vorteilhaft, wenn die Strömungswege für den polymerisationsfähigen Stoff durch die Heizeinrichtung hindurch ebenfalls parallel zur Schwerkraft verlaufen. Damit werden Bereiche innerhalb der Strömungswege vermieden, in denen es zu einer verstärkten Ansammlung des zugeleiteten Gases kommt. Die gleichmäßige Verteilung des Gases in dem polymerisationsfähigen Stoff bleibt über die gesamte Dauer des Durchströmens der Heizeinrichtung erhalten. Dadurch wird Polymerisation in der Heizvorrichtung deutlich reduziert und teilweise sogar dauerhaft verhindert.

Die Mittel zur gleichmäßigen Verteilung des Gases über den Einlass gewährleisten, dass jeder Teilstrom des polymerisationsfähigen Stoffes beim Durchströmen der Heizvorrichtung im wesentlichem mit dem gleichen Gehalt an Gas, insbesondere einem die Polymerisation hemmenden Gas, versorgt ist und somit in allen Strömungswegen des polymerisationsfähigen Stoffes die gleiche, geringe Neigung zur Polymerisation vorliegt. Die Mittel können z.B. mit besonders geeigneten Ausgestaltungen der Begasungsvorrichtung und/oder des Einlass und/oder separat ter Bauteile in der Anlage verwirklicht sein. Besonders bevorzugte Ausgestaltungen werden nachfolgend erläutert.

Die aufrechte Bauweise der Anordnung sowie die gleichmäßige Einbringung bzw. Verteilung des Gases in den polymerisationsfähigen Stoff reduziert in beachtlichen Umfang die Polymerisationsneigung, so dass die Heizvorrichtung über eine lange Zeitdauer mit hohem Wirkungsgrad arbeitet. Damit können in beachtlichen Umfang Kosten und der Wartungsaufwand reduziert werden.

Gemäß einer weiteren Ausgestaltung der Anordnung wird vorgeschlagen, dass die Heizvorrichtung mindestens einen senkrecht stehenden Rohrbündelwärmetauscher umfasst. Bei einem Rohrbündelwärmetauscher wird der polymerisationsfähige Stoff durch eine Vielzahl von Rohre hindurchgeführt, die von einem heißeren Medium (z.B. Wasserdampf) umströmt wird. Ein solcher Rohrbündelwärmetauscher ist vorzugsweise mit einer Höhe im Bereich von 4 bis 6 m ausgeführt. Die waagerechte Ausdehnung liegt beispielsweise im Bereich von 1 bis 2,50 m. Bevorzugt ist ein solcher Rohrbündelwärmetauscher in einem Kolonnen-ähnlichen Behälter untergebracht. Neben dem Rohrbündelwärmeaustauschern können auch Lamellenbündelwärmeaustauscher, welche enge Kanäle aufweisen, die aus Blechpaketen durch Punkt- oder Saumschweißen hergestellt sind, als Heizvorrichtung eingesetzt werden. Hinsichtlich des genauen Aufbaus von Rohrbündel- bzw. Lamellenbündelwärmeaustauschern wird auf "Grundoperationen Chemischer Verfahrenstechnik", Wilhelm R. A. Vauck und Hermann A. Müller, Wiley-VCH-Verlag, 11. überarbeitete und erweiterte Auflage, 2000, Seiten 502-506. verwiesen.

Die Begasungsvorrichtung ist in einem Abstand zum Einlass der Heizvorrichtung angeordnet, der in einem Bereich von 300 bis 1000 mm liegt. Bevorzugt ist ein Bereich von 300 bis 500 mm.

Damit wird eine besonders kompakte Anordnung geschaffen. Gerade im Zusammenspiel mit den Mitteln zur gleichmäßigen Verteilung des Gases kann trotz eines solch geringen Abstandes, eine sehr gute Verteilung des Gases gewährleistet werden. Die Begasungsvorrichtung mündet dabei z.B. nicht in eine Rohrleitung sondern in eine Art Mischkammer im Übergangsbereich von der Rohrleitung zur Heizvorrichtung. Besonders kleine Abstände können eingehalten werden, wenn die Begasungsvorrichtung entsprechend ausgeführt ist, also beispielsweise selbst bereits eine verteilte Zufuhr des Gases über mehrere Begasungsstellen ermöglicht.

Die Begasungsvorrichtung umfasst mindestens eine Düse, die einen Öffnungswinkel von mindestens 30° aufweist. Bevorzugt sind Düsen mit einem Öffnungswinkel im Bereich von 40° bis 50°. Dabei wird das Gas vorteilhafterweise mit einem Überdruck von mindestens 2 bar, bevorzugt im Bereich von 3 bar, in den flüssigen polymerisationsfähigen Stoff eingedüst. Im Hinblick auf die Positionierung der mindestens einen Düse sollte gewährleistet sein, dass eine gleichmäßige Begasung des Einlasses der Heizvorrichtung gewährleistet ist. So ist beispielsweise eine einzelne Düse möglichst zentrisch zum Einlass zu positionieren, während mehrere Düsen entweder über den Querschnitt des Einlass und/oder am Umfang gleichmäßig verteilt anzuordnen sind. Besonders bevorzugt ist, dass die Düsen so ausgerichtet sind, dass das Einströmen des Gases entgegen der Strömungsrichtung des polymerisationsfähigen Stoffes erfolgt.

Weiter ist vorteilhaft, dass zwischen der Begasungsvorrichtung und dem Einlass mindestens ein Strömungsbeeinflusser vorgesehen ist. Der Strömungsbeeinflusser hat insbesondere die Funktion, den flüssigen polymerisationsfähigen Stoff aufzuteilen bzw. abzulenken. Mögliche Ausgestaltungen eines Strömungsbeeinflussers sind beispielsweise ein Sieb, ein Gitter, ein Lochblech, etc.. Im Hinblick auf ein Lochblech ist besonders bevorzugt, dass dieses einen freien Strömungsquerschnitt im Bereich bis 30 % hat. Als Lochdurchmesser wird bevorzugt ein Bereich von 20 bis 30 mm angegeben. Der Strömungsbeeinflusser kann gegebenenfalls zumindest teilweise auch als Prallplatte für das Gas und/oder den polymerisationsfähigen Stoff dienen, so dass eine Verwirbelung bzw. Aufweitung von Teilströmungen stattfindet. Bevorzugt ist eine Ausgestaltung des Strömungsbeeinflussers, bei dem der gesamte Strom des polymerisationsfähigen Stoffes durch ihn hindurchströmt.

Nach einer weiteren Ausgestaltung der Anordnung ist in Strömungsrichtung des polymerisationsfähigen Stoffes gesehen vor der Begasungsvorrichtung mindestens ein Strömungskonditionierer zur Erzeugung einer Querströmung vorgesehen. Ein solcher Strömungskonditionierer wirkt bevorzugt primär (nur) auf den Strom des polymerisationsfähigen Stoffes. Er bewirkt eine Verwirbelung, Rotation oder ähnliches. Damit kann gewährleistet werden, dass sich der polymerisationsfähige Stoff im Bereich der Aufweitung vor dem Einlass der Heizvorrichtung schnell und gleichmäßig verteilt. Weiter ist auch möglich, dass ein gezieltes Vorbeiströmen des polymerisationsfähigen Stoffes an der Begasungsvorrichtung stattfindet. Weiter ist vorteilhaft, dass der Strömungskonditionierer über eine gewisse Länge in Strömungsrichtung eine Art Strömungsführung vornimmt. Diese Länge ist vorteilhafterweise nicht kleiner als 300 mm zu wählen und liegt bevorzugt im Bereich größer 500 mm.

In diesem Zusammenhang ist es besonders vorteilhaft, dass der mindestens eine Strömungskonditionierer mehrere Leitbleche zur Erzeugung einer Rotation zumindest eines Teils des polymerisationsfähigen Stoffes aufweist. Damit wird insbesondere bewirkt, dass gerade bei einem frontalen Anströmen des polymerisationsfähigen. Stoffes hin zum Einlass der Heizvorrichtung nun mittels dem Strömungskonditionierer eine Ablenkung in eine Querrichtung stattfindet.

Weiter wird ein Verfahren zur Erwärmung eines polymerisationsfähigen Stoffes vorgeschlagen, wobei der polymerisationsfähige Stoff in einer erfindungsgemäßen Anordnung zunächst mit einem Gas umfassend Sauerstoff angereichert und danach erwärmt wird.

Bei dem polymerisationsfähigen Stoff handelt es sich vorzugsweise um eine ethylenisch ungesättigte Verbindung ausgewählt aus der Gruppe bestehend aus Acrylsäure, Methacrylsäure, Acrylsäureestern und Methacrylsäureestern, wobei als Methacrylsäureester Methacrylsäuremethylester und Methacrylsäureethylester und als Acrylsäureester Acrylsäuremethylester und Acrylsäurethylester besonders bevorzugt sind. Am meisten bevorzugt handelt es sich bei dem polymerisationsfähigen Stoff um Acrylsäure.

Weiterhin ist es erfindungsgemäß bevorzugt, dass der polymerisationsfähige Stoff, in den das Gas eingeleitet wird, neben den vorstehend genannten ethylenisch ungesättigten Verbindungen weitere Nebenprodukte beinhaltet.

Gemäß einer besonderen Ausführungsform des erfindungsgemäßen Verfahrens basiert der polymerisationsfähige Stoff auf
(α1) 50 bis 99,5 Gew.-%, besonders bevorzugt 60 bis 99 Gew.-% (Meth)Acrylsäure,
(α2) 0,0001 bis 20 Gew.-%, vorzugsweise 5 bis 15 Gew.-% eines Trennmittels,
(α3) 0,0001 bis 5 Gew.-%, besonders bevorzugt 0,001 bis 1 Gew.-% Wasser,
(α4) 0,0001 bis 10 Gew.-%, besonders bevorzugt 0,5 bis 5 Gew.-% dimerer (Meth)Acrylsäure, sowie
(α5) 0,1 bis 15 Gew.-%, besonders bevorzugt 0,5 bis 10 Gew.-% weitere Nebenprodukte,
wobei die Summe der Komponenten (α1) bis (α5) 100 Gew.-% beträgt.

Im Falle der Behandlung von Acrylsäure als polymerisationsfähiger Stoff handelt es sich bei dem Trennmittel vorzugsweise um organische Lösungsmittel, welche mit Wasser ein Azeotrop bilden. Besonders bevorzugt als Trennmittel ist Toluol. Bei den Nebenprodukten handelt es sich im Falle der Behandlung von Acrylsäure vorzugsweise um Verbindungen ausgewählt aus der Gruppe umfassend, Protoanemonin, Propionsäure, Maleinsäure, Maleinsäureanhydrid, Aldehyde wie Furfural, Benzaldehyd oder Propionaldehyd und Polymerisationsinhibitoren wie Hydrochinon oder Phenothiazin.

Gemäß einer anderen besonderen Ausführungsform des erfindungsgemäßen Verfahrens basiert der polymerisationsfähige Stoff auf
(β1) 0 bis 1 Gew.-%, besonders bevorzugt 0,0001 bis 0,1 Gew.-% (Meth)Acrylsäure,
(β2) 50 bis 99 Gew.-%, besonders bevorzugt 60 bis 98 Gew.-% (Meth)Acrylsäureestern,
(β3) 0,0001 bis 1 Gew.-%, besonders bevorzugt 0,01 bis 0,5 Gew.-% Wasser,
(β4) 0 bis 2 Gew.-%, besonders bevorzugt 0,0001 bis 1 Gew.-% eines Alkohols, sowie
(β5) 0,5 bis 10 Gew.-%, besonders bevorzugt 1 bis 5 Gew.-% weitere Nebenprodukte,
wobei die Summe der Komponenten (β1) bis (β5) 100 Gew.-% beträgt.

Bei den Alkoholen handelt es sich vorzugsweise um einen primären Alkohol ausgewählt aus der Gruppe umfassend Methanol, Ethanol, 1-Propanol, 2-Propanol, n-Butanol, iso-Butanol oder tert.-Butanol, am meisten bevorzugt n-Butanol, und bei den Estern dementsprechend vorzugsweise um Methylester, Ethylester, Propylester oder Butylester. Als Nebenprodukte sind, neben Polymerisationsinhibitoren, beispielsweise Verbindungen ausgewählt aus der Gruppe bestehend aus di-n-Butylether, n-Butylacetat und Butylbutyrat zugegen.

Als Gas, welches in den polymerisationsfähigen Stoff eingeleitet wird, sind Sauerstoff oder sauerstoffhaltige Gasgemische wie beispielsweise Luft bevorzugt.

Gemäß einer besonderen Ausführungsform des erfindungsgemäßen Verfahrens zur Erwärmung eines polymerisationsfähigen Stoffes handelt es sich bei dem polymerisationsfähigen Stoff um die Zusammensetzung, die als Sumpfprodukt nach folgendem Verfahren erhalten wird:
A) katalytische Gasphasenoxidation von Propylen unter Bildung eines Acrolein enthaltenden ersten Produktgasgemisches;
B) katalytische Gasphasenoxidation des Acroleins unter Bildung eines Acrylsäure enthaltenden zweiten Produktgasgemisches;
C) Kondensation des zweiten Produktgasgemisches in einem Quenchtower unter Erhalt einer wässrigen Acrylsäurelösung;
D) azeotrope Destillation der wässrigen Acrylsäurelösung in Gegenwart von Toluol als Schleppmittel unter Erhalt eines Sumpfproduktes und Kopfproduktes.

Gemäß einer besonderen Ausgestaltung des erfindungsgemäßen Verfahrens wird der mit Sauerstoff angereicherte polymerisationsfähige Stoff auf eine Temperatur von mindestens 85°C, besonders bevorzugt von mindestens 90°C erwärmt. Üblicherweise strömt der polymerisationsfähige Stoff, wenn es sich hierbei um einen wiederaufbereiteten aus einer Destillationskolonne handelt, mit einer Temperatur von ca. 70 bis 80° an. Gemäß dem hier vorgeschlagenen Verfahren kann nun eine Temperaturerhöhung auf bis zu 91°C erreicht werden, was unter Berücksichtigung der kurzen Verweildauer innerhalb der Heizvorrichtung beachtlich ist. Die Strömungsgeschwindigkeit des polymerisationsfähigen Stoffes beträgt beispielsweise 2 bis 5 m/s. Dies macht einen besonders guten Wärmeübergang in der Heizvorrichtung erforderlich, was mit den vorstehend genannten Maßnahmen sichergestellt werden kann, denn Polymerisation behindert den Wärmeübergang nicht mehr wesentlich.

Dabei ist bevorzugt, dass der polymerisationsfähige Stoff mit einem gemittelten Volumenstrom von 500 bis 3000 m³/h zugeführt wird. Dies hat beachtliche wirtschaftliche Konsequenz, da beispielsweise die Herstellung von Acrylsäure sehr schnell und in großem Umfang durchgeführt werden kann.

Gerade bei der Heizeinrichtung, die im Zusammenwirken mit einer Destillationsvorrichtung betrieben wird, wird im Hinblick auf die vorstehend genannten Volumenströme vorgeschlagen, dass das Sauerstoff umfassende Gas mit einem gemittelten Massenstrom von 60 bis 180 kg/h zugeführt wird. Diese Menge eines die Polymerisation hemmenden Gases ist einerseits ausreichend zur Vermeidung von Polymerisation in der Heizvorrichtung und andererseits klein bemessen, da aufgrund der gleichmäßigen Verteilung eine Überversorgung nicht erforderlich ist.

Die vorliegende Erfindung betrifft auch eine Vorrichtung zur Herstellung von hochreiner (Meth)Acrylsäure, umfassend als fluidleitend miteinander verbundene Vorrichtungsbestandteile
(a) einen Synthesereaktor,
(b) eine Absorptions- oder Kondensationsvorrichtung, sowie
(c) eine Aufreinigungsvorrichtung,
wobei die Aufreinigungsvorrichtung (c) mindestens eine erfindungsgemäße Anordnung zur Behandlung eines polymerisationsfähigen Stoffes umfasst.

Als Reaktor (a) können alle dem Fachmann bekannten Reaktoren eingesetzt werden, die üblicherweise zur Herstellung von (Meth)Acrylsäure eingesetzt werden. Bevorzugte Reaktoren sind Rohrbündelreaktoren mit einem Katalysator-Festbett sowie Reaktoren, welche mit Katalysator beschichtete Thermobleche oder Thermobleche mit einer Katalysator-Festbettschüttung aufweisen. Die Reaktoren weisen neben dem mit dem Katalysator in Verbindung stehenden Reaktionsraum auch einen von einem Kühlmedium durchströmten Wärmetransportraum auf. Erfindungsgemäß bevorzugte Reaktoren sind beispielsweise in EP-A-0 700 893 sowie in DE-A-101 08 380 beschrieben.

Bei der Absorptions- oder Kondensationsvorrichtung (b) handelt es sich vorzugsweise um einen sogenannten "Quenchtower", wie er in der EP-A-1 319 648 beschrieben ist. In einem solchen Quenchtower wird die in der zweiten Oxidationsstufe (in der (Meth)Acrolein zur (Meth)Acrylsäure oxidiert wird) erhaltene (Meth)Acrylsäure sowie das bei der Oxidation entstehende Reaktionswasser einer Totalkondensation unter Bildung einer wässrigen (Meth)Acrylsäurelösung unterworfen. Grundsätzlich bevorzugte Kondensationsvorrichtungen sind Kondensatoren mit trennwirksamen Einbauten, insbesondere mit Packungen, Füllkörpern und/oder. Böden, vorzugsweise Glockenböden, Siebböden, Ventilböden und/oder Dual-Flow-Böden. Dabei werden die kondensierbaren Komponenten des gasförmigen Produktgemisches fraktioniert durch Kühlung auskondensiert. Da das Gasgemisch infolge der Verunreinigungen und Verdünnungsgase eine Schwersieder-, Mittelsieder- und Leichtsiederfraktion sowie nichtkondensierbare Komponenten enthält, können bei der Kolonne an den entsprechenden Stellen ein oder mehrere Seitenabzüge vorgesehen sein. Im Gegensatz zu einer üblichen Kondensation ermöglicht eine Kondensation in einer Kolonne somit bereits eine Auftrennung in die einzelnen Komponenten. Geeignete Kolonnen umfassen wenigstens eine Kühlvorrichtung, wofür sich alle gängigen Wärmeübertrager oder Wärmetauscher, bei denen die bei der Kondensation gebildete Wärme indirekt (extern) abgeführt wird, eignen. Bevorzugt sind Rohrbündelwärmetauscher, Plattenwärmetauscher und Luftkühler. Geeignete Kühlmedien sind Luft bei dem entsprechenden Luftkühler und Kühlflüssigkeiten, insbesondere Wasser, bei anderen Kühlvorrichtungen. Ist nur eine Kühlvorrichtung vorgesehen, so wird diese am Kopf der Kolonne eingebaut, in dem die Leichtsiederfraktion auskondensiert wird. Da das (Meth)Acryl-säurehaldge Gasgemisch mehrere Fraktionen enthält, ist es zweckmäßig, mehrere Kühlvorrichtungen in verschiedenen Abschnitten der Kolonne einzubauen, z.B. eine Kühlvorrichtung im unteren Abschnitt der Kolonne zur Auskondensation der Schwersiederfraktion und eine Kühlvorrichtung am Kopf der Kolonne zur Auskondensation der Leichtsiederfraktion. Im Falle der Herstellung von Acrylsäure wird diese in einer Fraktion im mittleren Teil der Kolonne über einen oder mehrere Seitenabzüge abgezogen.

Neben der Kondensation der gasförmigen Reaktionskomponenten können diese auch in einer mit trennwirksamen Einbauten versehenen Absorptionskolonne innig mit einer Absorptionsflüssigkeit in Kontakt gebracht und auf diese Weise absorbiert werden.

Die im Quenchtower erhaltene wässrige Acrylsäurelösung, die in der Kondensationskolonne vorzugsweise als Seitenstrom abgezogene Acrylsäure oder die nach Absorption in einem geeigneten Lösungsmittel erhaltene Acrylsäurelösung wird anschließend in einer geeigneten Aufreinigungsvorrichtung (c) weiter aufgereinigt. Diese Aufreinigungsvorrichtung (c) umfasst mindestens eine erfindungsgemäße Anordnung zur Behandlung eines polymerisationsfähigen Stoffes. Gemäß einer besonderen Ausführungsform der erfindungsgemäßen Vorrichtung von (Meth)Acrylsäure umfasst die Aufreinigungsvorrichtung (c) eine Destillationsvorrichtung, die dergestalt mit einer erfindungsgemäßen Anordnung verbunden ist, dass zumindest ein Teil des Sumpfproduktes der Destillationsvorrichtung kontinuierlich mittels eines Förderelementes, vorzugsweise mittels einer Pumpe, aus der Destillationsvorrichtung entfernt, in einer erfindungsgemäßen Anordnung behandelt, das heißt mit einem Gas in Kontakt gebracht und erhitzt wird, und anschließend in die Destillationsvorrichtung zurückgeführt wird.

Gemäß einer besonderen Ausführungsform der erfindungsgemäßen Vorrichtung handelt es sich bei der Kondensationsvorrichtung (b) um einen Quenchtower und bei der Aufreinigungsvorrichtung (c) um eine Destillationskolonne, in der in Gegenwart von Toluol als Schleppmittel die im Quenchtower erhaltene wässrige Acrylsäurelösung azeotrop destilliert wird.

Die Erfindung betrifft auch ein Verfahren zur Herstellung hochreiner (Meth)acrylsäure umfassend die Verfahrensschritte:
(A) Herstellen eines (Meth)Acrylsäure enthaltenden Produktgasgemisches in einem Reaktor,
(B) Aufreinigung des Produktgasgemisches unter Erhalt einer (Meth)Acrylsäure mit einer Reinheit von mindestens 99,5 Gew.-%, besonders bevorzugt mindestens 99,8 Gew.-% und darüber hinaus bevorzugt mindestens 99,9 Gew.-% in einer Aufreinigungsvorrichtung umfassend mindestens eine erfindungsgemäße Anordnung.

Gemäß dem Verfahrensschritt (A) wird zunächst in einem erfindungsgemäßen Reaktor ein (Meth)Acrylsäure enthaltendes Produktgasgemisch hergestellt. Vorzugsweise werden dabei zwei in Reihe geschaltete Reaktoren eingesetzt, wobei im ersten Reaktor die Oxidation von Propylen bzw. Isobutylen oder anderen geeigneten Ausgangsverbindungen zu (Meth)Acrolein und im zweiten Reaktor die Umsetzung von (Meth)Acrolein zur (Meth)Acrylsäure erfolgt. Denkbar ist jedoch auch, die Oxidation zur (Meth)Acrylsäure in einem erfindungsgemäßen Reaktor einstufig durchzuführen.

Im Verfahrensschritt (B) erfolgt die Aufreinigung des (Meth)Acrylsäure enthaltenden Produktgasgemisches. Dabei wird mindestens ein bei der Aufreinigung der (Meth)Acrylsäure anfallender, (Meth)Acrylsäure enthaltender Produktstrom in einer erfindungsgemäßen Vorrichtung behandelt. Die Aufreinigung umfasst dabei vorzugsweise mindestens einen Destillationsschritt.

Die vorliegende Erfindung betrifft auch die durch das vorstehend beschriebene Verfahren erhältliche hochreine (Meth)Acrylsäure.

Zudem betrifft die Erfindung ein Verfahren zur Herstellung eines (Meth)Acrylsäure beinhaltenden Polymers, wobei eine durch das vorstehend beschriebene Verfahren erhältliche, hochreine Meth)Acrylsäure polymerisiert wird. Die Polymerisation erfolgt vorzugsweise als Lösungspolymerisation, wobei die Reaktionsführung in einem Muldenband besonders bevorzugt ist. Hierbei wird entweder die wässrige Phase unmittelbar eingesetzt oder die wasserarme Phase entsprechend verdünnt. Im allgemeinen erfolgt die Polymerisation in einem Medium mit einem Wassergehalt von 20 bis 80 Vol.-%, bezogen auf das Medium.

Außerdem betrifft die Erfindung ein Polymer, erhältlich nach dem im vorstehenden Absatz beschriebenen Verfahren.

Bei dem Polymer handelt es sich vorzugsweise um ein Absorbierendes Polymer mit einer maximale Aufnahme von 0.9 Gew.-%iger wässriger NaCl-Lösung gemäß ERT 440.1-99 in einem Bereich von 10 bis 1000, bevorzugt von 15 bis 500 und besonders bevorzugt von 20 von 300 ml/g. Weitere Einzelheiten zu absorbierenden Polymeren und deren Herstellung ergeben sich aus *"*Modern Superabsorbent Polymer Technology" Fredric L. Buchholz, Andrew T. Graham, Whiley-VCH, 1998.

Die erfindungsgemäße hochreine (Meth)Acrylsäure oder das erfindungsgemäße Polymer wird in oder zur Herstellung von Fasern, Formkörpern, Filmen; Schäumen, superabsorbierenden Polymeren oder Hygieneartikeln verwendet.

Die Erfindung sowie das technische Umfeld werden nachfolgend anhand der Figuren näher erläutert, ohne dass die Erfindung darauf begrenzt wäre. Es ist darauf hinzuweisen, dass die in den Figuren veranschaulichten Größenverhältnisse nicht die tatsächlichen Größenverhältnisse darstellen, es sei denn, in der Figurenbeschreibung wird explizit darauf hingewiesen. Es zeigen:
- Fig. 1:: schematisch eine erste Ausführungsvariante einer erfindungsgemäßen Anordnung,
- Fig. 2:: schematisch ein Detail einer weiteren Ausführungsvariante der Anordnung,
- Fig. 3:: eine Ausführungsvariante eines Strömungsbeeinflussers,
- Fig. 4:: eine weitere Ausgestaltung der Anordnung im Detail,
- Fig. 5:: eine schematische Darstellung einer Ausführungsvariante eines Strömungskonditionierers,
- Fig. 6:: schematisch einen Teil der (Meth)Acrylsäure-Herstellung, und
- Fig. 7:: schematisch ein Strömungsprofil durch einen Strömungskonditionierer.

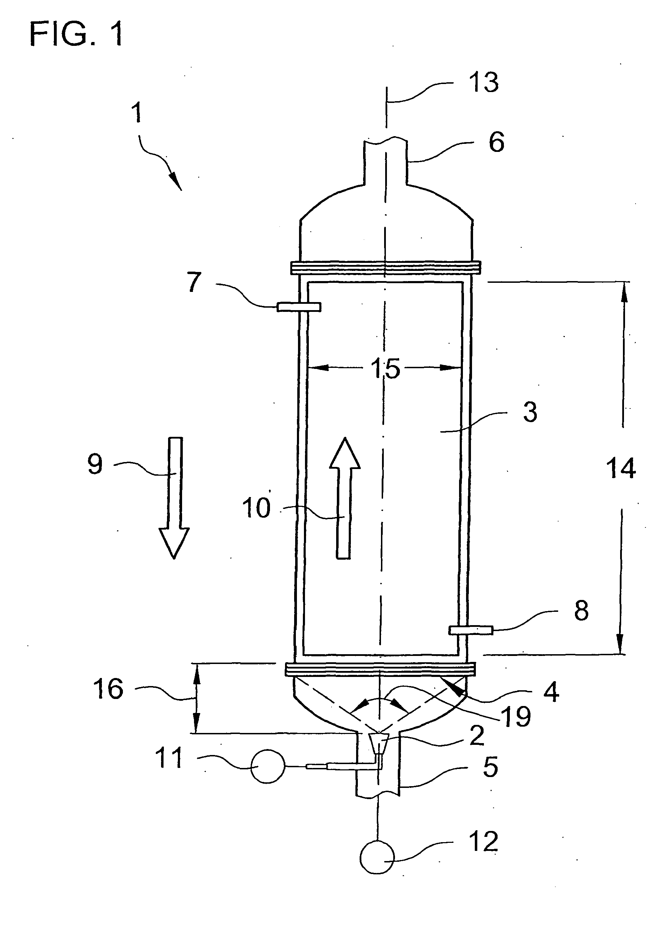

Fig. 1 zeigt eine Anordnung 1 zur Behandlung eines polymerisationsfähigen Stoffes 12 mit einer Begasungsvorrichtung 2 zum Einleiten eines Gases 11 in den polymerisationsfähigen Stoff 12 und eine Heizvorrichtung 3 zum Erhitzen des mit Gas 11 versehenen polymerisationsfähigen Stoffes 12. Die Heizvorrichtung 3 ist so gestaltet, dass der polymerisationsfähige Stoff 12 die Heizvorrichtung 3 über einen Einlass 4 entgegen der Schwerkraft 9 durchströmt.

Der polymerisationsfähige Stoff 12 strömt als Flüssigkeit durch den Edukteintritt 5 an der Begasungsvorrichtung 2 vorbei. Die Begasungsvorrichtung 2 ist hier mit einem zentrisch zur Achse 13 angeordneten Düsensystem ausgeführt. Die Begasungsvorrichtung 2 verteilt das Gas 11 (insbesondere einen Sauerstoff umfassenden Polymerisationsinhibitor) mit einem Öffnungswinkel 19 in dem flüssigen, polymerisationsfähigen Stoff 12. Dabei ist die Begasungsvorrichtung 2 in einem solchen Abstand 16 zum Einlass 4 der Heizvorrichtung 3 positioniert, dass der Öffnungswinkel 19 den gesamten Querschnitt des Einlass 4 erreicht. Damit sind geeignete Mittel zur gleichmäßigen Verteilung des Gases in dem polymerisationsfähigen Stoff angegeben.

Der mit dem Gas 11 angereicherte Strom des polymerisationsfähigen Stoffes 12 tritt nun in die Heizvorrichtung 3 ein und durchströmt sie mit der Strömungsrichtung 10, die im wesentlichen parallel und entgegengesetzt zur Gravitation 9 (Schwerkraft) verläuft. Innerhalb der Heizvorrichtung 3 wird der polymerisationsfähige Stoff 12 in getrennten Strömungswegen hin zum Produktaustritt 6 geleitet. Das Aufheizen erfolgt über ein dampfförmiges Medium, welches über einen Dampfeintritt 7 zu- und über einen Kondensataustritt 8 abgeführt wird. Damit ergibt sich eine Aufwärmstrecke, die im wesentlichen der Höhe 14 der Heizvorrichtung 3 entspricht. Üblicherweise wird eine solche Heizvorrichtung 3, insbesondere wenn sie als Rohrbündelwärmetauscher ausgeführt ist, mit einer Ausdehnung 15 im Bereich von 500 mm bis 2500 mm ausgeführt.

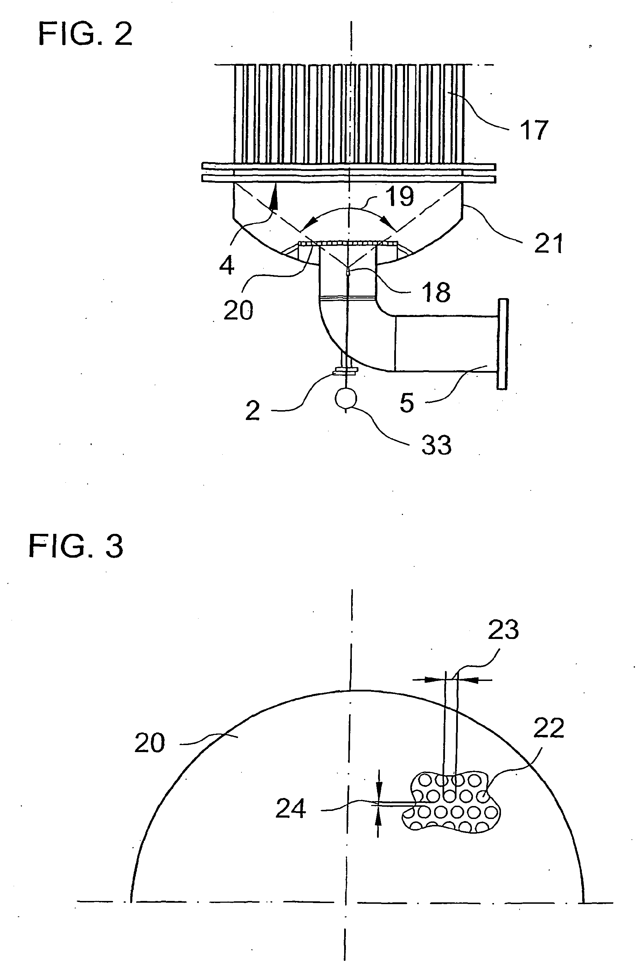

Fig. 2 zeigt nun ein Detail einer weiteren Ausgestaltung einer Anordnung, bei welcher der Edukteintritt 5 gebogen und in der Krümmung eine Zufuhr für die Begasungsvorrichtung 2 vorgesehen ist. Die Begasungsvorrichtung 2 verteilt wiederum das Gas 11 in dem polymerisationsfähigen Stoff 12, wobei der Öffnungswinkel 19 so bemessen ist, dass der Einlass 4 eingeschlossen ist. Hierfür wird die Begasungsvorrichtung 2 mit einem Förderer 33 für das Gas 11 betrieben, der einen Überdruck im Inneren des Edukteintritts 5 bzw. der Mischkammer 21 von mindestens 2 bar gewährleistet. Die Begasungsvorrichtung 2 weist eine einzelne Düse 18 auf, die bevorzugt als Vollkegeldüse ausgeführt und zentrisch positioniert ist.

Stromabwärts der Düse 18 durchströmen der polymerisationsfähige Stoff 12 und das Gas 11 zunächst einen Strömungsbeeinflusser 20, der als Lochblech ausgeführt ist. Der Strömungsbeeinflusser 20 bewirkt eine Verwirbelung von Teilströmen nach Austritt aus dem Strömungsbeeinflusser 20, so dass eine gleichmäßige Verteilung des Gas-Stoff-Gemischs stattfindet. Somit ist im wesentlichen die gleiche Konzentration des Gases 11 in den Rohren des Rohrbündelwärmetauschers 17 sichergestellt.

Eine Teilansicht eines solchen Strömungsbeeinflussers 20 ist in Fig. 3 dargestellt. Der Strömungsbeeinflusser 20 weist eine Vielzahl von Öffnungen 22 auf, die beispielsweise eine Erstreckung 23 von ca. 25 mm aufweisen. Die Öffnungen 22 sind beispielsweise in einer solchen Entfernung 24 zueinander zu positionieren, dass eine gleichmäßige Verteilung der Öffnungen 22 gegeben ist, wobei die Summe der Öffnungen 22 etwa 30 % der gesamten Oberfläche des Strömungsbeeinflussers 20 ausmachen.

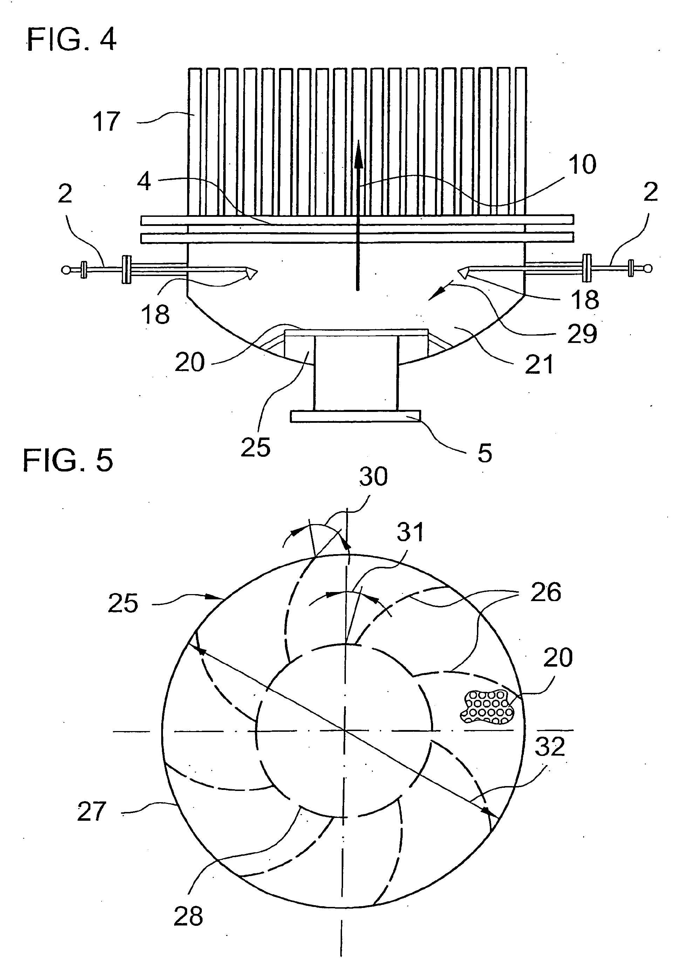

Fig. 4 veranschaulicht eine weitere Ausführungsvariante einer Anlage 1 mit mehreren Begasungsvorrichtungen 2. Hierbei strömt der polymerisationsfähige Stoff 12 zunächst über den Edukteintritt 5 in die Mischkammer 21 vor dem Einlass 4 des Rohrbündelwärmetauschers 17. Im Übergang vom Edukteintritt 5 hin zur Mischkammer 21 ist ein Strömungsbeeinflusser 25 vorgesehen. Dieser bewirkt, dass zumindest Teile des einströmenden polymerisationsfähigen Stoffes 12 hin zu den Begasungsvorrichtungen 2 abgelenkt werden. Damit strömt der polymerisationsfähige Stoff 12 entgegen der Begasungsrichtung 29 und eine gleichmäßige Verteilung kann erreicht werden.

Fig. 5 zeigt ein Ausführungsbeispiel eines solchen Strömungskonditionierers 25. Dieser ist mit einem Außenzylinder 27 und einem Innenzylinder 28 aufgebaut, zwischen denen eine Mehrzahl von Leitblechen 26 vorgesehen sind. Die Leitbleche 26 sind mit einem Außenwinkel 30 an dem Außenzylinder 27 und einem Innenwinkel 31 an dem Innenzylinder 28 befestigt. Zum Ablenken der Strömung in radiale Richtung weist der Strömungskonditionierer 25 einen Strömungsbeeinflusser 20 als eine Art "Deckel" auf. Der damit erzeugte Staudruck bewirkt, dass sich Teile des Stromes an den Leitblechen 26 entlang bewegen und auf diese Weise zum Rotieren angeregt werden. Üblicherweise hat ein solcher Strömungskonditionierer 25 einen Durchmesser 32 der ausreichend groß ist, den Edukteintritt 5 zu überdecken.

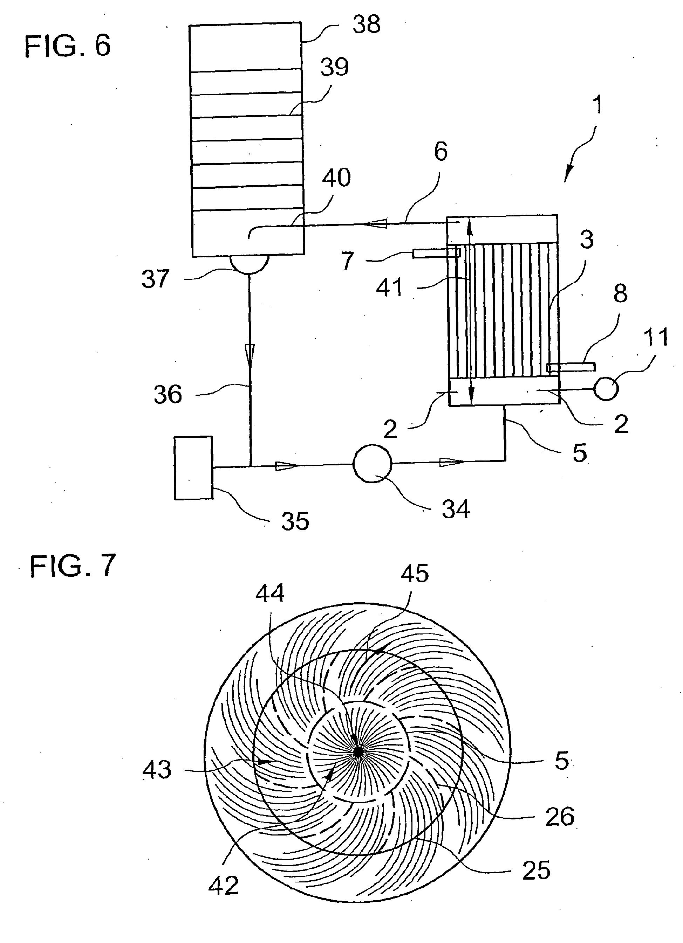

Fig. 6 veranschaulicht den Stofftransport durch die Anlage 1 und eine nachfolgend angeordnete Destillationskolonne 38, wie es bei der Herstellung von Acrylsäure üblich ist. Der polymerisationsfähige Stoff 12 wird mittels einer Pumpe 34 und mit einer Temperatur im Bereich von ca. 80°C über den Edukteintritt 5 der Begasungsvorrichtung 2 des Gases 11 (Inhibitor) zugeführt. Anschließend durchströmt das Gas-Stoff-Gemisch eine Heizvorrichtung 3. Die gesamte Anordnung 1 hat dabei eine Dimension 41 von ca. 10 m. Mit einer solchen Anordnung 1 werden besonders hohe Volumenströme des polymerisationsfähigen Stoffes 12 in sehr kurzer Zeit auf Temperaturen beim Produktaustritt 6 im Bereich von 90° erwärmt. Die Verweilzeit des polymerisationsfähigen Stoffes liegt dabei in Abhängigkeit von der Dimension 41 der Anlage 1 in Bereichen unterhalb von 10 Sekunden. Das erhitzte, noch flüssige Gas-Stoff-Gemisch strömt nun hin zur Destillationskolonne 38 und wird dort über einen Zulauf 40 eingeleitet. Dabei entspannt sich die Flüssigkeit und steigt als Dampf nach oben durch die einzelnen Trennböden 39 der Destillationskolonne 38. Das Kondensat sammelt sich in einem Sammelbecken 37 der Destillationskolonne 38 und wird über einen Abfluss 36 wieder der Pumpe 34 zugeführt. Dieser Kreislauf kann auch mit einem Reservoir 35 ergänzt werden, der bei Bedarf zusätzlichen polymerisationsfähigen Stoff 12 bereitstellt.

Fig. 7 soll die Strömungsverhältnisse in einem Strömungskonditionierer 25 veranschaulichen. Der Strömungskonditionierer 25 ist mit seinen Leitblechen 26 eingezeichnet. Zentrisch ist der Edukteintritt 5 gestrichelt gekennzeichnet. Die im zentralen Bereich einströmende Menge des polymerisationsfähigen Stoffes 12 wird durch den oben angebrachten Strömungsbeeinflusser 20 (hier nicht dargestellt) zumindest teilweise abgelenkt und hin zu den Leitblechen 26 geführt. Deshalb lässt sich in der Darstellung in Fig. 7 in Zentrumnähe ein Ruhebereich 44 ausmachen, während am Randbereich des Edukteintritts 5 die Normalströmung 42 ausgebildet ist. Beim Durchströmen des Strömungskonditionierers 25 bzw. der Leitbleche 26 erfolgt eine Strömungsbeschleunigung 43, was durch dunkle Bereiche symbolisiert wird. Gleichzeitig erfolgt eine Rotation 45 des polymerisationsfähigen Stoffes 12. Dies hat eine besonders gleichmäßige Bewegung des polymerisationsfähigen Stoffes 21 in der Mischkammer zur Folge, so dass hier der Polymerisationsinhibitor besonders gut verteilt eingebracht werden kann.

### Bezugszeichenliste

- 1: Anordnung
- 2: Begasungsvorrichtung
- 3: Heizvorrichtung
- 4: Einlass
- 5: Edukteintritt
- 6: Produktaustritt
- 7: Dampfeintritt
- 8: Kondensataustritt
- 9: Gravitation
- 10: Strömungsrichtung
- 11: Gas
- 12: Stoff
- 13: Achse
- 14: Höhe
- 15: Ausdehnung
- 16: Abstand
- 17: Rohrbündelwärmetauscher
- 18: Düse
- 19: Öffnungswinkel
- 20: Strömungsbeeinflusser
- 21: Mischkammer
- 22: Öffnung
- 23: Erstreckung
- 24: Entfernung
- 25: Strömungskonditionierer
- 26: Leitblech
- 27: Außenzylinder
- 28: Innenzylinder
- 29: Begasungsrichtung
- 30: Außenwinkel
- 31: Innenwinkel
- 32: Durchmesser
- 33: Förderer
- 34: Pumpe
- 35: Reservoir
- 36: Abfluss
- 37: Sammelbecken
- 38: Destillationskolonne
- 39: Trennboden
- 40: Zulauf
- 41: Dimension
- 42: Normalströmung
- 43: Strömungsbeschleunigung
- 44: Ruhebereich
- 45: Rotation

## Patentansprüche

1. Anordnung (1) zur Behandlung eines polymerisationsfähigen Stoffes (12) umfassend zumindest eine Begasungsvorrichtung (2) zum Einleiten eines Gases (11) in den polymerisationsfähigen Stoff (12) und eine Heizvorrichtung (3) zum Erhitzen des mit Gas (11) versehenen polymerisationsfähigen Stoffes (12), bei der die Heizvorrichtung (3) so gestaltet ist, dass der polymerisationsiahige Stoff (12) die Heizvorrichtung (3) über einen Einlass (4) entgegen der Schwerkraft (9) durchströmt und Mittel zur gleichmäßigen Verteilung des Gases (11) über den Einlass (4) vorgesehen sind, wobei die mindestens eine Begasungsvorrichtung (2) in einem Abstand (16) zum Einlass (4) der Heizvorrichtung (3) angeordnet ist, der in einem Bereich von 300 bis 1000 mm liegt und die Begasungsvorrichtung (2) weiter mindestens eine Düse (18) umfasst, die einen Öffnungswinkel (19) von mindestens 30° aufweist.

2. Anordnung (1) nach Anspruch 1, **dadurch gekennzeichnet, dass** die Heizvorrichtung (3) mindestens einen senkrecht stehenden Rohrbündelwärmetauscher (17) umfasst.

3. Anordnung (1) nach einem der vorhergehenden Ansprüche, **dadurch gekennzeichnet, dass** zwischen der Begasungsvorrichtung (2) und dem Einlass (4) mindestens ein Strömungsbeeinflusser (20) vorgesehen ist.

4. Anordnung (1) nach einem der vorhergehenden Ansprüche, **dadurch gekennzeichnet, dass** in Strömungsrichtung (10) des polymerisationstahigen Stoffes (12) gesehen vor der Begasungsvorrichtung (2) mindestens ein Strömungskonditionierer (25) zur Erzeugung einer Querströmung vorgesehen ist.

5. Anordnung nach Anspruch 4, **dadurch gekennzeichnet, dass** der mindestens eine Strömungskonditionierer (25) mehrere Leitbleche (26) zur Erzeugung einer Rotation zumindest eines Teils des polymerisationsfähigen Stoffes (12) aufweist.

6. Verfahren zur Erwärmung eines polymerisationsfähigen Stoffes (12), wobei der polymerisationsfähige Stoff (12) in einer in einem der Ansprüche 1 bis 5 definierten Anordnung (1) zunächst mit einem Gas (11) umfassend Sauerstoff angereichert und danach erwärmt wird.

7. Verfahren nach Anspruch 6, wobei der mit Sauerstoff angereicherte polymerisationsfähige Stoff (12) auf eine Temperatur von mindestens 80°C erwärmt wird.

8. Verfahren nach Anspruch 6 oder 7, wobei der polymerisationsfähige Stoff (12) (Meth)Acrylsäure ist.

9. Verfahren nach einem der Ansprüche 6 bis 8, **dadurch gekennzeichnet, dass** der polymerisationsfähige Stoff (12) mit einem gemittelten Volumenstrom im Bereich von 500 bis 3000 m³/h zugeführt wird.

10. Verfahren nach einem der Ansprüche 6 bis 9, **dadurch gekennzeichnet, dass** das Sauerstoff umfassende Gas (11) mit einem gemittelten Massenstrom von 60 bis 180 kg/h zugeführt wird.

11. Eine Vorrichtung zur Herstellung von hochreiner (Meth)Acrylsäure, umfassend als fluidleitend miteinander verbundene Vorrichtungsbestandteile
(a) einen Reaktor,
(b) eine Absorptions- oder Kondensationsvorrichtung, sowie
(c) eine Aufreinigungsvorrichtung
wobei die Aufreinigungsvorrichtung (c) mindestens eine Anordnung nach einem der Ansprüche 1 bis 5 umfasst.

12. Ein Verfahren zur Herstellung hochreiner (Meth)acrylsäure umfassend die Verfahrensschritte:
(A) Herstellen eines (Meth)Acrylsäure enthaltenden Produktgasgemisches in einem Reaktor,
(B) Aufreinigung des Produktgasgemisches unter Erhalt einer (Meth)Acrylsäure mit einer Reinheit von mindestens 99,5 Gew.-% in einer Aufreinigungsvorrichtung umfassend mindestens eine Anordnung nach einem der Ansprüche 1 bis 5.

## Claims

1. Assembly (1) for the treatment of a polymerisable material (12) comprising at least one gassing device (2) for introducing a gas (11) into the polymerisable material (12) and a heating device (3) for heating the polymerisable material (12) provided with gas (11), in which the heating device (3) is configured in such a way that the polymerisable material (12) flows through the heating device (3) via an inlet (4) against gravity (9) and means are provided for uniform distribution of the gas (11) over the inlet (4), wherein the at least one gassing device (2) is arranged at a distance (16) from the inlet (4) of the heating device (3) lying in a range of from 300 to 1,000 mm and the gassing device (2) further comprises at least one nozzle (18) having an opening angle (19) of at least 30°.

2. Assembly (1) according to claim 1, **characterised in that** the heating device (3) comprises at least one perpendicularly positioned shell-and-tube heat exchanger (17).

3. Assembly (1) according to one of the preceding claims, **characterised in that** at least one flow influencer (20) is provided between the gassing device (2) and the inlet (4).

4. Assembly (1) according to one of the preceding claims, **characterised in that** at least one flow conditioner (25) is provided, before the gassing device (2) viewed in the flow direction (10) of the polymerisable material (12), for generating a transverse flow.

5. Assembly according to claim 4, **characterised in that** the at least one flow conditioner (25) has a plurality of deflectors (26) for generating rotation of at least a part of the polymerisable material (12).

6. Process for heating a polymerisable material (12), wherein the polymerisable material (12) is first enriched with a gas (11) comprising oxygen and then heated in an assembly (1) defined in one of claims 1 to 5.

7. Process according to claim 6, wherein the polymerisable material (12) enriched with oxygen is heated to a temperature of at least 80°C.

8. Process according to claim 6 or 7, wherein the polymerisable material (12) is (meth)acrylic acid.

9. Process according to one of claims 6 to 8, **characterised in that** the polymerisable material (12) is supplied at an average volume flow in the range of from 500 to 3,000 m³/h.

10. Process according to one of claims 6 to 9, **characterised in that** the gas (11) comprising oxygen is supplied at an average mass flow of from 60 to 180 kg/h.

11. A device for preparing high-purity (meth)acrylic acid, comprising as device components connected to one another in a fluid-conveying manner:
(a) a reactor,
(b) an absorption or condensation device, and also
(c) a purification device,
wherein the purification device (c) comprises at least one assembly according to one of claims 1 to 5.

12. A process for preparing high-purity (meth)acrylic acid, including the following process steps:
(A) preparing a (meth)acrylic acid-containing product gas mix in a reactor,
(B) purifying the product gas mix so as to obtain a (meth)acrylic acid having a purity of at least 99.5 % by weight in a purification device comprising at least one assembly according to one of claims 1 to 5.

## Revendications

1. Arrangement (1) pour le traitement d'une substance polymérisable (12), comprenant au moins un dispositif d'introduction de gaz (2) pour l'introduction d'un gaz (11) dans la substance polymérisable (12), et un dispositif de chauffage (3) pour le chauffage de la substance polymérisable (12) comportant le gaz (11), dans lequel le dispositif de chauffage (3) se présente de sorte que la substance polymérisable (12) traverse le dispositif de chauffage (3) par une admission (4) contre la force de gravité (9) et dans lequel des moyens pour la distribution homogène du gaz (11) sont présents, au moins un dispositif d'introduction de gaz (2) étant disposé à une distance (16) de l'admission (4) du dispositif de chauffage (3), qui se situe dans l'intervalle allant de 300 à 1000 mm et le dispositif d'introduction de gaz (2) comprenant en outre, au moins une buse (18), qui présente un angle d'ouverture (19) d'au moins 30°.

2. Arrangement (1) selon la revendication 1, **caractérisé en ce que** le dispositif de chauffage (3) comprend au moins un échangeur de chaleur à faisceau tubulaire (17) vertical.

3. Arrangement (1) selon l'une des revendications précédentes, **caractérisé en ce qu'**entre le dispositif d'introduction de gaz (2) et l'admission (4), est présent au moins un modificateur de courant (20).

4. Arrangement (1) selon l'une des revendications précédentes, **caractérisé en ce que** dans la direction d'écoulement (10) de la substance polymérisable (12), envisagée avant le dispositif d'introduction de gaz (2), est présent au moins un appareil de conditionnement du courant (25) pour la production d'un courant transversal.

5. Arrangement (1) selon la revendication 4, **caractérisé en ce que** au moins un appareil de conditionnement de courant (25) présente plusieurs tôles de guidage (26) pour la production d'une rotation d'au moins une partie de la substance polymérisable (12).

6. Procédé de chauffage d'une substance polymérisable (12), où la substance polymérisable (12) est d'abord enrichie d'un gaz (11) comprenant de l'oxygène et est ensuite chauffée, dans un arrangement (1) tel que défini dans l'une des revendications 1 à 5.

7. Procédé selon la revendication 6, où la substance polymérisable (12) enrichie d'oxygène est chauffée à une température d'au moins 80°C.

8. Procédé selon la revendication 6 ou 7, où la substance polymérisable (12) est l'acide (méth)acrylique.

9. Procédé selon l'une des revendications 6 à 8, **caractérisé en ce que** la substance polymérisable (12) est alimentée avec un courant volumique moyen situé dans l'intervalle allant de 500 à 3000 m³/heure.

10. Procédé selon l'une des revendications 6 à 9, **caractérisé en ce que** le gaz comprenant de l'oxygène (11) est alimenté avec un courant massique moyen situé dans l'intervalle allant de 60 à 180 kg/heure.

11. Dispositif pour la préparation d'acide (méth)acrylique très pur, comprenant comme constituants du dispositif, reliés l'un à l'autre en communication fluide,
(a) un réacteur,
(b) un dispositif d'absorption ou de condensation, ainsi que
(c) un dispositif de purification.
où le dispositif de purification (c) comprend au moins un arrangement selon l'une des revendications 1 à 5.

12. Procédé de préparation d'acide (méth)acrylique très pur, comprenant les étapes de procédé :
(A) Préparation d'un mélange gazeux de produit contenant l'acide (méth)acrylique dans un réacteur,
(B) Purification du mélange gazeux de produit avec obtention d'acide (méth)acrylique ayant une pureté d'au moins 99,5% en poids dans un dispositif de purification comprenant au moins un arrangement selon l'une des revendications 1 à 5.
